(19) Europäisches Patentamt European Patent Office Office européen des brevets

(11) **EP 4 205 712 B1**

(12) **EUROPEAN PATENT SPECIFICATION**

(45) Date of publication and mention of the grant of the patent:
**30.04.2025   Bulletin 2025/18**

(21) Application number: 21383228.0

(22) Date of filing: **28.12.2021**

(51) International Patent Classification (IPC):
***A61F 13/02*** *(2024.01)*

(52) Cooperative Patent Classification (CPC):
**A61F 13/0263; A61F 13/0203; A61F 13/0209**

(54) **ATRAUMATIC ABSORBENT WOUND DRESSING**

ATRAUMATISCHER ABSORBIERENDER WUNDVERBAND

PANSEMENT ABSORBANT POUR BLESSURES ATRAUMATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.07.2023   Bulletin 2023/27**

(73) Proprietor: **Paul Hartmann AG
89522 Heidenheim (DE)**

(72) Inventors:
• **GALVAN, Gemma
08013 Barcelona (ES)**
• **GONZÀLEZ, Fernando
08030 Barcelona (ES)**
• **MOSQUERA, Óscar
73431 Aalen (CO)**
• **ECKSTEIN, Axel
89522 Heidenheim (DE)**

(74) Representative: **Paul Hartmann AG
Patents & Licensing
Paul-Hartmann-Straße 12
89522 Heidenheim (DE)**

(56) References cited:
EP-A1- 3 281 617        EP-A1- 3 669 843
WO-A1-2014/097069   WO-A1-2018/217621
WO-A1-2020/051548   WO-A1-93/19710

## Description

### Field of the Invention

The present invention relates to a self-adhesive, atraumatic, absorbent wound dressing. In particular, the present invention relates to a wound dressing which may remain on a wound for an extended period of time.

### Background of the Invention

Atraumatic and absorbent wound dressings as described in EP3281617 A1, EP3669843 A1 or WO 93/19710 A1 are available for the treatment of wounds. The atraumatic properties allow a painless and in ideal cases non-irritating removal of the wound dressing. Due to absorbent features of such dressings excess exudate can be removed from the wound and collected within the dressing. The atraumatic properties are usually conveyed by inert and hydrophobic substances like silicone or silicone-based adhesives. Apertures in the layers contacting the wound are needed for the removal of the exudate. However, such apertures may reduce the atraumatic properties of the wound contacting layer because the growing wound tissue may penetrate into the holes and adhere to absorbent layers of the wound dressing.

When using silicone-based adhesives a further challenge is the reduced adhesive strength when the dressing is worn for an extended period of time. Although the adhesive forces of silicone-adhesives are usually suitable for a secure application of the wound dressing over a short period of time, during application periods extending more than just a few days, non-irritating adhesives may lose proper connection to the skin. This effect is even more evident when the non-irritating adhesive is bent or subjected to movement or vibration. This is for example the case when the wound dressing is attached to moving areas of the body such as joints.

A decrease in adhesive strength is even more problematic when the patient is bedridden and dependent on a so-called anti-decubitus mattresses. Usually, the affected patient group is in an advanced age. The skin of these patients often is thin, dry and shows reduced elasticity. The respective areas are referred to as parchment skin. The use of conventional and possibly stronger adhesives on such skin is not indicated, as pain or injuries may occur during dressing changes. The anti-decubitus mattresses stimulate a change of body position, using, for example, air pumps inside the mattress. However, the forces acting on the patient reduce the risk of decubitus but have a negative impact on the retention of non-irritating silicone adhesives.

Some atraumatic, absorbent wound dressings are usually designed as so-called island wound dressings. As the dressing covers the whole wound area including the intact skin, the wound is shielded from external influences and bacterial colonization. This effect is only guaranteed as long as the edge of the wound dressing adheres firmly to the skin. Complete or partial detachment of the dressing from the skin provides an entry point for pathogens which may infect the wound. Once the wound has been infected the microorganisms may spread further. In case of post-surgery wounds or deep cavities an infection of organs or sepsis may occur. It is therefore of outermost importance that a detachment of the wound dressing is avoided. As a countermeasure a frequent dressing change is possible. However, this disturbs the wound healing process, it is unpleasant for the patient, and it also consumes caregivers' resources. In addition, every change of the dressing comes along with a short period during which the uncovered wound is exposed to the environment and may get infected.

The object of the invention is to further improve wound healing. In particular, the object of the present invention is to provide an atraumatic, absorbent wound dressing which prevents the ingrowth of newly formed tissue into the absorbent material of the dressing and reliably adheres to the skin, even when the affected area of the body is subjected to movement or vibration thereby leading to a wound dressing which may be worn over an extended period of time. At the same time the wound dressing should be capable to rapidly absorb fluids exudating from the wound, even if they occur in large quantities.

### Summary of the Invention

The invention provides an improved atraumatic absorbent wound dressing as well as a method for the production of this dressing.

The provided wound dressing comprises

- a backing layer comprising a nonwoven material,
- an absorbent wound pad comprising super absorbent fibers and a net layer, the net layer being directed towards the wound side,
- a perforated atraumatic wound contact layer comprising a polyurethane layer, an adhesive acrylic layer and an adhesive silicone layer as skin adherent, wherein the polyurethane layer is located between the adhesive acrylic layer and the adhesive silicone layer and wherein the perforated atraumatic wound contact layer and the backing layer extend beyond the wound pad thereby creating an edge region and wherein the backing layer and the perforated atraumatic wound contact layer are bonded together at the edge region.

The inventive method for manufacturing an absorbent wound dressing comprises the steps of:

    a) Providing a backing layer comprising a nonwoven material
    b) Attaching an absorbent wound pad comprising i) a layer having super absorbent fibers and ii) a net layer to the backing layer, whereby the net layer is directed towards the wound side,

    c) Providing a polyurethane layer coated with an adhesive acrylic layer on one side and coated with an adhesive silicone layer on the other side wherein the polyurethane layer, the adhesive acrylic layer and the adhesive silicone layer are perforated and

    d) Attaching the polyurethane layer with the adhesive acrylic layer towards the backing layer and the wound pad, whereby the adhesive silicone layer is to be oriented in a way that it can be placed on a wound.

The inventors of the present invention discovered that it is advantageous to combine a further atraumatic net layer with an atraumatic wound contact layer, whereby the wound contact layer has an adhesive silicone as skin adherent. The net layer constitutes a component of the wound pad. Using the inventive combination of layers, the ingrowth of tissue from the wound through the apertures of the wound contact layer into the absorbent material is prevented or at least reduced. At the same time the arrangement proposed according to the invention allows to provide a wound dressing capable of absorbing high amounts of exsudates within a reasonable time. The dressing may remain on a wound site for several days, for example between 2 and 7 days, even if the patient moves or is exposed to vibrations.

A further advantage of the present invention is the improved adhesive strength appearing in the edge region of the wound dressing. Thereby, the attachment of the wound dressing to the skin is improved, even under influence of movement- or vibration forces. The risk of an undesired contamination of the wound is reduced.

### Detailed Description of the Invention

In the present application the terms proximal side or proximal surface are to be understood as that side or surface of a layer or material of the wound dressing that is in use facing a wound of a patient. Accordingly, a surface or layer which can be placed on a wound or which is directed towards the wound side is proximal. The terms distal side or distal surface are to be understood as that side or surface of a layer or material that is in use facing away from the wound.

The terms pad, wound pad, absorbent pad or absorbent wound pad are used interchangeably. In addition, the terms aperture and perforation are used interchangeably.

Flexibility within the meaning of this application is the property that enables a material to bend and to change its conformation. It is characterized by the stiffness.

Flexibility is important for the dressing as a non-flexible dressing is very stiff and causes pressure on the patient's body surface during movement resulting in discomfort or pain.

The invention presented herein provides an absorbent atraumatic dressing having a flexibility, which is sufficient to adapt the dressing to an irregular body surface and to allow for a movement of the patient without causing undesired discomfort.

The backing layer of the proposed dressing comprises a nonwoven material. The backing layer may have any shape, such as square, rectangular, circular, oval, trapezium-shaped, suitably with rounded corners.

The backing layer supports the absorbent pad. It provides a barrier to the passage of microorganisms into the dressing. According to a preferred aspect of the invention the backing layer is substantially liquid-impermeable but permeable for water vapour. Preferably the backing layer has a moisture vapor transmission rate (MVTR) from 400 to 20000 $g/m^2/24h$, suitably 600 to 14000 $g/m^2/24h$ according to test method DIN EN 13726-2. In a more preferred embodiment the moisture vapor transmission rate is between 800 to 12000 $g/m^2/24h$ according to test method DIN EN 13726-2.

The backing layer preferably has a thickness of 0.16 mm to 1 mm, preferably 0.3 mm to 0.85 mm, more preferably 0.4 mm to 0.75 mm and most preferably 0.45 mm to 0.71 mm.

The backing layer comprises a proximal surface and a distal surface. The distal surface of the backing layer may be a low-friction surface in order to exhibit advantageous wearing properties without sticking to clothes or causing noise during use.

Suitable polymers for forming the nonwoven backing layer material include polyurethanes, poly alkoxyalkyl acrylates, methyl acrylates such as those disclosed in GB1280631 and nonwoven like polyester nonwoven and especially spunlace polyester nonwoven.

According to one aspect of the invention the nonwoven backing material is hydrophobic and / or water-repellant. In this context, all polymers in which non-polar properties predominate are understood to be water repellent. Among these are aliphatic polymers as well as polymers which consist of or comprise polyester and / or polyurethane. The advantage of a hydrophobic and / or water-repellant backing is that the dressing and the wound is shielded from water or other fluids that are applied to the dressing from the outside. A backing layer having strong hydrophobic and / or water-repellant properties may allow the patient to wear the wound dressing while taking a shower without irritating or infecting the wound.

The backing layer which is hydrophobic and / or water-repellant may still provide an adequate MVTR. The backing layer may for example be designed as a membrane having microperforations.

The backing layer may be transparent in order to allow a view of the wound pad positioned on the proximal side of the backing layer. This allows visual control of the wound pad. In this way, it is possible to check how much the wound pad is filled with exudate.

Also, the colour of the exudate may be inspected. An unusual color of the exudate indicates an infection of the wound and allows the medical staff to initiate countermeasures. A backing layer which is transparent may furthermore be hydrophobic and / or water-repellant as described herein.

According to a preferred embodiment an additional layer of an adhesive is present between the backing layer and the wound pad, wherein the additional layer of adhesive preferably extends beyond the wound pad. More preferably the adhesive is an acrylic adhesive. Most preferably the additional layer of adhesive acrylic is made of acrylic based hot-melt adhesive. According to an even more preferred embodiment, the additional layer of adhesive is coated to the proximal surface of the backing layer. The backing layer may be coated with an adhesive on the proximal surface. The adhesive secures the backing layer to other layers of the wound dressing, particularly to the absorbent pad. If the additional layer of adhesive, in particular acrylic adhesive, extends beyond the wound pad it may also advantageously be used to bond the backing layer and to the wound contact layer. According to this particularly advantageously embodiment, the adhesive which is coated on the proximal surface of the backing layer may act as an additional layer of adhesive in addition to the adhesive silicone layer in the wound contact layer, as those areas of the backing layer which are oriented above the perforations in the wound contact layer can also cause adhesive bonding to the skin through the perforations. The backing layer together with the layer of adhesive acrylic coated thereon may have essentially the same thickness as the backing layer without the adhesive acrylic. The inventors discovered that the additional adhesive effect caused by the adhesively coated backing reaching through said perforations of the wound contact layer improves the durability of the adhesive fixation of the dressing when exposed to vibration forces. This effect is particularly strong when an acrylic-based adhesive is used. It was found that there is a synergistic effect caused by the interaction of the acrylic adhesive coated to the backing in combination with the silicon adhesive applied used as a skin adherent. In general, suitable adhesives for coating the proximal surface of the backing include pressure-sensitive adhesives like acrylic acid, acrylate ester copolymers, polyvinyl ethyl ether and polyurethane. Further information on suitable adhesives can be found in GB1280631. Silicone-based adhesives are not recommended for coating the proximal surface of the backing layer, however, a small amount of silicone may be used as long as the required adhesive properties are maintained.

The basis weight of the additional adhesive is suitably 10 $g/m^2$ to 100 $g/m^2$, preferably 15 $g/m^2$ to 50 $g/m^2$, more preferably 30 $g/m^2$ to 40 $g/m^2$ and most preferably 34 $g/m^2$ to 38 $g/m^2$.

The backing layer can be coated with adhesive continuously, i.e. the adhesive covers the entire or essentially the entire proximal surface of the backing layer.

In different embodiments, the backing layer can be coated partially with the additional layer of adhesive. In these embodiments the adhesive can be in the form of stripes, dots or in different patterns.

In a further embodiment the backing layer comprises an adhesive layer disposed on the proximal surface, whereby an adhesive-free part in the center of the backing layer is arranged to reduce contact with the distal surface of the wound pad. According to a preferred embodiment the edge region of the proximal surface of the backing layer is fully covered with the additional layer of adhesive while the center of the backing layer, which overlaps with the wound pad is coated partially.

The MVTR in an adhesive-free region of the backing layer is increased. If the absorbent core within the absorbent pad swells after absorption of wound exudate the absorbent pad changes its spatial extension. This can either cause delamination due to the occurrence of shearing forces within the wound dressing, or it can cause stress and pain on the wound and/or the wound surrounding skin due to shearing forces acting on the wound and skin of the patient. A dressing leaving an adhesive-free area between the distal surface of the absorbent pad and the proximal surface of the backing layer can advantageously reduce said stress and pain on the wound.

According to a preferred embodiment the backing layer is a nonwoven backing which is coated on its proximal side with 34 $g/m^2$ to 38 $g/m^2$ acrylic adhesive or acrylic based hot-melt adhesive.

The absorbent pad comprises absorbent material including superabsorbent fibers. The absorbent material should be hydrophilic material which is chemically inert towards water. It should be capable of absorbing wound exudate. Preferably the absorbent material contains superabsorbent particles and fibers. The absorbent material may be a superabsorbent material as described herein. While superabsorbent fibers cause a quick absorption of wound exudate additionally present superabsorbent particles may exhibit a high absorption capacity.

Suitable materials for absorbent fibers include cellulose or cellulose-based polymers, viscose, polyester, polyamide, derivatives, copolymers and mixtures thereof.

Superabsorbent fibers can be based on polyacrylic acid, sodium polyacrylate (also known as sodium acrylate), polyacrylic acid esters or copolymers thereof. A mixture of these three compounds is preferred. Even more preferred is a crosslinked polymer composed of these three compounds. At most preferred are superabsorbent fibers comprising or consisting of such crosslinked polymer.

According to a preferred embodiment of the invention, the wound pad comprises non-woven fibers made from viscose, polyethylene and polypropylene, and superabsorbent fibers.

The superabsorbent fibers can be loosely distributed within the wound pad or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials. Preferably the textile material is a first nonwoven layer as described herein.

In a preferred embodiment the absorbent pad and / or absorbent core exhibits an absorption capacity of at least 100 g/100 cm$^2$, preferably more than 120 g/100 cm$^2$, more preferably more than 130 g/100 cm$^2$ according to the test method described as Example 3 within the example part of this application.

The superabsorbent material can be present in an amount of from 1 % by weight to 99 % by weight within the absorbent pad or in the absorbent core. Preferably, the amount of superabsorbent material comprises from 30 % to 55 % by weight of the absorbent pad or the absorbent core.

A superabsorbent material is generally understood to be a water-insoluble, swellable polymer that can absorb a multiplicity of its own weight of a liquid such as water, saline solutions, or body fluids. The absorption of liquid results in the formation of a hydrogel. The absorption capacity for pure water is typically greater than the absorption capacity for saline liquid. In connection with this invention, the term superabsorbent material refers, in particular, to a material exhibiting a an absorption capacity (free expansion) according to the standard test method DIN EN 13726-1:2002 Section 3.2 of at least 20 g/g, preferably at least 30 g/100cm$^2$ and most preferably at least 40 g/100cm$^2$, wherein the fluid handling capacity according to the same test method Section 3.3 is at least 12 g/100cm$^2$/24h, preferably at least 14 g/100cm$^2$/24h and most preferably at least 16 g/100cm$^2$/24h. The retention of exudate according to test method TM00 0152 is at least 91 %, preferably at least 93 %, more preferably at least 95 % and most preferably at least 97 %.

The thickness of the wound pad is correlated with the absorption capacity. Suitable ranges of the thickness of the wound pad (including the absorbing material) are 1 mm to 10 mm, 2 mm to 8 mm, 2.5 mm to 6 mm and 2.8 mm to 4 mm. If a thinner layer is used, more wound dressings will fit into one packing carton. Also, the gas exchange in the area of the wound and the adjacent skin is improved. A thicker layer will be able to absorb higher amounts of wound exudate. Surprisingly, it has turned out that the use of super absorbent fibers including a mixture of sodium acrylate (or sodium polyacrylate), acrylic acid and methyl acrylate allows compact dimensions of the wound pad in the range of 2.8 mm to 4 mm and enables absorption of even larger amounts of wound exudate such as occurring in postoperative injuries. Such a wound dressing may stay on a strong exudating wound over a prolonged time of at least 5 days. Depending on the specific wound such a dressing may even stay for at least 8 days continuously on the wound.

The wound pad furthermore comprises a net layer at its proximal side. The net layer acts as an additional atraumatic layer (in addition to the atraumatic wound contact layer) and improves the non-irritating properties of the wound dressing. Using dressings described in the prior art, wound tissue may grow through the apertures in the wound contact layer The dressing according to the invention reduces this undesired effect, whereby the synergistic interaction between the perforated atraumatic wound contact layer and the novel net layer seems to be responsible. In the absence of the net layer the wound tissue could get in direct contact with the fibers of the wound pad and granulation tissue could grow into the wound pad. This would become more likely the longer the dressing stays on the wound. If tissue were allowed to grow through the apertures of the wound contact layer into the wound pad an atraumatic removal of the wound dressing would be hindered or even impossible.

The net layer on the proximal side of the wound pad can be made of various materials. Preferably the net layer is made from a polymer. Preferably the net layer or the polymer from which it is made is chemically inert against any interaction with the components contained in the wound exudate. The net layer or the polymer from which it is made must not dissolve in contact with water. It was found that polyethylene is especially suitable. Therefore, according to one aspect of the invention the wound pad is covered on its proximal side with a net layer comprising or consisting of polyethylene.

According to one embodiment of the invention the wound pad comprises a first nonwoven layer and the super absorbent fibers are integrated in this first nonwoven layer. This first nonwoven layer may comprise cotton and/or viscose, polyethylene and polypropylene. A blend including one, two, three or all of these compounds is possible. Preferably the first nonwoven layer comprises or consists of viscose, polyethylene and polypropylene.

Preferably, the absorbent material is integrated as super absorbent fibers into the first nonwoven layer. In this context it is recommended to use a first nonwoven layer consisting of or comprising viscose, polyethylene and polypropylene. The quantity ratio for the first nonwoven layer made of viscose, polyethylene and polypropylene with the super absorbent fibers integrated therein, may be selected as follows: 50 % to 90 % (mass percentage) viscose, polyethylene and polypropylene and 10 % to 50 % super absorbent fibers, preferably 60 % to 85 % viscose, polyethylene and polypropylene and 15 % to 40 % super absorbent fibers, more preferably 70 % to 80 % viscose, polyethylene and polypropylene and 20 % to 30 % super absorbent fibers.

Furthermore, the wound pad preferably comprises a second nonwoven layer, wherein the second nonwoven layer is located between the first nonwoven layer and the net layer. Preferably, the second nonwoven layer does not comprise any superabsorbent material such as superabsorbent fibers or particles. The first and / or the second nonwoven layer may comprise one or more materials selected from the group consisting of: viscose, cotton, tencell, modal, rayon, polyethylene and polypropylene. Preferably the first nonwoven layer comprises or consists of an air-laid non-woven. The air-laid non-woven may comprise or consist of one or more of the materials listed in this paragraph. Preferably the second nonwoven layer comprises viscose. The second nonwoven is an exudate transmission layer. When using the wound dressing the exudate from the wound flows through the wound contact layer, the net layer and is directed by the exudate transmission layer into the absorbent material of the wound pad where it is retained. The advantage is a rapid and reliable uptake of wound exudate in a unilateral direction. This process may also support the removal of microorganisms like bacteria from the wound in case the wound got infected. The microorganisms are taken up and retained together with the wound exudate.

Preferably, the exudate transmission layer comprises a blend of viscose, polyethylene and polypropylene. Preferred is a transmission layer comprising 50 % to 99 % (percentage in relation to the mass) viscose and 1 % to 50 % polyethylene and polypropylene, more preferred 60 % to 95 % viscose and 5 % to 40 % polyethylene and polypropylene, most preferred 70 % to 90 % viscose and 10 % to 30 % polyethylene and polypropylene. The inventors of the present invention found that a blend of 85 % viscose and 15 % polyethylene and polypropylene is particularly well suited. The transmission layer has absorbent properties, but these are weaker than those of the absorbent material which retains the exudate.

Preferably, the first and the second nonwoven layers are in direct contact with each other, so that no additional material is located between the first and the second nonwoven layer. It was found that placing the layers in direct contact supports fluid uptake from the wound.

The purpose of the wound pad is to absorb and retain wound exudate. Aptly, the wound pad has an absorption capacity (free expansion) according to the standard test method DIN EN 13726-1:2002 Section 3.2 of preferably at least 10 g/g, more preferably at least 20 g/g, especially preferred at least 30 g/100 cm$^2$ and most preferably at least 40 g/100 cm$^2$ wherein the fluid handling capacity according to the same test method Section 3.3 is at least 12 g/100 cm$^2$/24 h, preferably at least 14 g/100 cm$^2$/24 h and most preferably at least 16 g/100 cm$^2$/24 h.

The perforated wound contact layer intended to be used for a wound dressing according to the invention comprises polyurethane layer, an adhesive acrylic layer and an adhesive silicone layer as skin adherent. The polyurethane layer is located between the adhesive acrylic layer and the adhesive silicone layer wherein the adhesive acrylic layer is located distal and the adhesive silicone layer proximal. The silicone adhesive provides for an atraumatic adhesion to the skin surrounding the wound site.

The adhesive acrylic which is located at the distal surface of the polyurethane layer is a pressure sensitive adhesive (PSA). Preferably, it is a translucent PSA acrylic adhesive. The amount of acrylic adhesive within the adhesive acrylic layer of the wound contact layer may be within the range of 5 g/m$^2$ to 100 g/m$^2$, preferably 10 g/m$^2$ to 80 g/m$^2$, more preferably 20 g/m$^2$ to 60 g/m$^2$, and most preferably 25 g/m$^2$ to 40 g/m$^2$.

Suitably, the polyurethane layer has a thickness of 1 $\mu$m to 2 mm, preferably 5 $\mu$m to 50 $\mu$m.

The polyurethane layer is a unitary sheet such as a polymer mesh, an perforated polymer film or a perforated polyurethane membrane. Polyurethan is highly flexible and chemically inert with respect to biomolecules. It does not dissolve in water. It may be stored for a long time without losing its properties.

The polyurethane layer may have a wide range of thickness of for example 1 $\mu$m to 2 mm, preferably 1.5 $\mu$m to 1 mm, more preferably 2 $\mu$m to 0.5 mm and most preferably 2.3 $\mu$m to 0.1 mm. Surprisingly, it has been found that a polyurethane film or a polyurethane membrane having a thickness in the range of 25 $\mu$m provides sufficient stability. This applies even to a polyurethane layer having perforations as disclosed herein. Such thin layers of polyurethane have the advantage of conforming well to the skin surface and being virtually silent when the patient moves. Furthermore, the incorporation of a polyurethane layer improves both the stability of the wound contact layer and its atraumatic properties.

The wound contact layer comprises on its proximal surface a layer of skin-friendly non-irritating silicone adhesive. The silicone composition is a so-called soft skin adhesive silicone elastomer. The total coating weight of the silicone is suitably from about 15 g/m$^2$ to about 500 g/m$^2$, preferably 50 g/m$^2$ to 250 g/m$^2$, more preferably 100 g/m$^2$ to 200 g/m$^2$ and most preferably 130 g/m$^2$ to 170 g/m$^2$.

The perforations of the wound contact layer completely penetrate the wound contact layer. Accordingly, the apertures extend from the proximal side of the adhesive silicone layer to the proximal side of the wound pad. As mentioned above the proximal side of the wound pad comprises a net layer.

The wound dressing according to the invention is designed as an island dressing, in which the absorbent pad is sandwiched between the backing and the wound contact layer. The perforated atraumatic wound contact layer and the backing layer extend beyond the wound pad and create an edge region. The backing layer and the perforated atraumatic wound contact layer are bonded together at the edge region. The advantage of an island dressing is that the wound is completely shielded from harmful environmental influences like for example dust, dirt and pathogenic microorganisms. Moreover, the dressing is self-adhesive and therefore fast and simple to apply.

Accordingly, to one aspect of the invention the perforated atraumatic wound contact layer and the backing layer of the wound dressing extend both in length and width beyond the wound pad such that the edge region builds a continuous margin around the wound pad. The continuous margin comprises the wound contact layer and the backing layer but not the wound pad. According to a preferred embodiment the continuous margin consists of the wound contact layer and the backing layer. A backing layer as part of the continuous margin may be covered on its proximal surface with an additional adhesive layer as described herein.

The dressing exhibits skin-friendly properties that allow the dressing to be removed without causing pain to the patient. The undesired irritation of the wound or the skin surrounding the wound is reduced. It has been found that a dressing exhibiting a peel strength on Bristol paper at an angle of 180° of 2 N/25mm to 3 N/25mm, more preferably 2.2 N/25mm to 2.8 N/25mm, most preferably 2.4 N/25mm to 2.7 N/25mm shows highly suitable properties with regard to skin adhesion. Measurement of peel strength is done according to FINAT N° 1.

The wound contact layer comprises apertures for the passage of wound exudate to the absorbent core. Apertures can have any geometrical shape, regular or irregular, in particular a circular, oval, triangular, square, rectangular, pentangular, hexagonal or other polygonal shape with uniform or non-uniform side length.

In a preferred embodiment the wound contact layer has an open area of 3 % to 25 % of the entire area of the wound contact layer. Preferably the open area is 5 % to 20 %, more preferably 10 % to 18 % and most preferably 12 % to 16 % of the entire area of the wound contact layer. It has been found that an open area in this range results in a favorable absorption velocity of wound exudate. Absorption velocity within the meaning of this application means the time of the absorption of wound exudate. It can be determined by a test method described in the example part of this application as Example 2.

In a preferred embodiment the wound contact layer comprises apertures of any of said shapes having an average open area of from 0.05 mm$^2$ to 8.0 mm$^2$. In a preferred embodiment the wound contact layer comprises apertures having an essentially circular shape and an average diameter of between 0.1 mm to 2.8 mm. It has been found that the absorption velocity is improved if the wound contact layer comprises apertures having a circular shape with an average diameter of 2.0 mm to 2.8 mm, preferably 2.2 mm to 2.6 mm. The apertures extend through the entire wound contact layer as described herein.

In the edge region of the wound dressing the apertures of the wound contact layer extend to the backing layer. If the proximal surface of the backing layer is coated by an adhesive acrylic as described above the adhesive is exposed to the skin of the patient through the apertures in the edge region. This allows a stronger adhesion of the wound dressing in the edge region to the skin surrounding the wound. Accordingly, the adhesive forces of the non-irritating adhesive like silicone adhesive are enhanced and complemented with the stronger adhesive forces of the additional layer of adhesive which attaches the backing layer directly on the skin through the apertures of the wound contact layer. The two different adhesives complement each other in a surprisingly good way. Via the additional layer of adhesive, the wound dressing according to the invention shows excellent resistance towards vibrations and moving forces (e.g., during sleep) while at the same time maintaining atraumatic and non-irritating properties. The resulting wound dressing according to the invention is highly suitable to be worn over an extended period of time as described herein. At the same time, the wound dressing according to the invention shows improved adhesion properties, when applied to freshly disinfected skin surface even if residues of disinfection agent remain on the skin.

The wound tissue is covered with the wound pad only. Therefore, the additional adhesive layer in the edge region cannot stick to the wound, but only to the skin surrounding the wound.

By adapting the diameter of the apertures provided in the wound contact layer the person skilled in the art may adjust the adhesive properties of the wound dressing as well as the non-irritating properties of the wound dressing. The larger the diameter of the apertures in the wound contact layer, the more skin will be covered with the additional layer of adhesive resulting in an increased resistance to vibration and movement.

Suitably, the open area of the apertured layer is from about 12 % to 27 %. Surprisingly it has been found that the absorption velocity is favorable for a wound contact layer having an open area of 12 % to 24 %, preferably 13 % to 20 %. Suitably, the density of the apertures is from about 10,000 to 200,000 apertures per m$^2$, for example about 15,000 to 175,000 apertures per m$^2$.

It has been found that a wound dressing according to the invention exhibits advantageous adhesive properties. A wound dressing according to the invention adheres to the skin without losing adhesiveness, which allows a use of the dressing for a prolonged time which might be at least three days, preferably for at least five days and more preferably at least seven days.

It has been found that the wound dressing according to the invention exhibits advantageous properties with regard to the absorption velocity.

The wound dressing in its entirety as well as the backing layer, the absorbent core, the absorbent pad and the wound contact layer each can have any suitable size and basic geometrical shape, particularly squares, rectangles, circles, ovals, polygons. If the basic geometrical shape of any of these layers comprises corners, these corners preferably are rounded. This reduces the risk of curling of the layer and of delamination from other layers or skin. In a preferred embodiment, the radius for the rounded corners is from 5 mm to 15 mm, more preferably 12.5 mm.

A high bond strength is advantageous to prevent undesired separation or delamination of the joined materials or joint layers. According to a preferred embodiment of the invention two layers of adhesive stick together within the edge region of the wound dressing. Thereby, the backing layer and the wound contact layer are attached in a strong and reliable manner to each other and resists vibration and movement. It has turned out that this construction is highly suitable to avoid delamination even if the wound dressing is worn over an extended period of time. A preferred example is a coating of an acrylic based hotmelt adhesive on the proximal surface of the backing layer and a coating of translucent PSA acrylic adhesive on the distal surface of the sheet layer within the wound contact layer.

A wound dressing according to the invention shows excellent flexibility and can therefore be placed on uneven body areas and joints while a secure attachment is provided by the two adhesives in the edge region of the dressing.

The dressing according to the present invention may further comprise at least one removable cover sheet to cover the adhesive areas on the proximal surface of the wound dressing. The cover sheet covers and protects the absorbent pad and prevents premature adhesion of the adhesive parts of the wound dressing. It may comprise a film of polyethylene, polypropylene or fluorocarbons and paper coated with these materials or silicone. A suitable material for a cover sheet can be a polyethylene foil having a thickness of 100 $\mu$m and can be purchased from the company Flextrus®.

A further aspect of the invention is a method for the manufacturing or production of an absorbent wound dressing comprising an adhesive atraumatic wound contact layer and having the properties and the structure described herein. The method comprises the following steps:

a) Providing a backing layer comprising a nonwoven material
b) Attaching an absorbent wound pad comprising i) a layer having super absorbent fibers and ii) a net layer to the backing layer, whereby the net layer is directed towards the wound side,
c) Providing a polyurethane layer coated with an adhesive acrylic layer on one side and coated with an adhesive silicone layer on the other side wherein the polyurethane layer, the adhesive acrylic layer and the adhesive silicone layer are perforated and
d) Attaching the polyurethane layer with the adhesive acrylic layer towards the backing layer and the wound pad, whereby the adhesive silicone layer is to be oriented in a way that it can be placed on a wound.

The net layer is on the proximal surface of the wound pad while the layer having super absorbent fibers is on the distal side of the wound pad or forms a part of the wound pad.

The adhesive silicone layer is located on the proximal surface of the polyurethane layer, while the adhesive acrylic layer is located on the distal surface of the polyurethane layer.

The steps of attaching may be performed by using adhesive forces of the adhesively coated surfaces, by welding, laminating or further suitable approaches.

**Examples**

Example 1: Wound dressing

The wound dressing has a backing layer, a wound pad, and a wound contact layer. The backing layer has a thickness of 0.58 mm and is made from hydrophobic spunlace polyester nonwoven. On its distal surface it is fully coated with an additional layer of Acrylic based hotmelt adhesive in an amount of 36 g/m$^2$. This additional layer of adhesive is between the backing layer and the wound pad and extends beyond the wound pad to the very end of the backing layer. The wound pad has a thickness of 2.9 mm and comprises super absorbent fibers and a net layer made of polyethylene on its proximal side. The super absorbent fibers are integrated in a first air-laid non-woven. Between the first nonwoven layer and the net layer there is a second nonwoven layer (an exudate transmission layer). The first and the second nonwoven layers are both part of the wound pad. The net layer provides the proximal end of the wound pad. The wound contact layer has a thickness of 25 $\mu$m and is formed from a clear PU film. This PU film is coated on its proximal surface with a non-irritating, translucent silicone adhesive in an amount of 150 g/m$^2$. On the distal surface the PU film is coated with translucent PSA acrylic adhesive in an amount of 30 g/m$^2$. The complete wound contact layer (including the acrylic adhesive, the PU film and the silicone adhesive) is perforated. The apertures extend through the whole wound contact layer. The apertures have a circular shape and an average diameter of 2.4 mm. The apertures are 5.9 mm apart from each other in one dimension and 2.55 mm in the other dimension so that they build up a regular pattern resulting in an open area of 15 % within the wound contact layer. The backing layer and the wound contact layer extend beyond the wound pad. Accordingly, there is a continuous margin around the wound pad that builds the edge region of the wound dressing. Within the edge region the additional layer of acrylic based hotmelt adhesive and the layer of translucent PSA stick together to connect the backing layer to the wound contact layer.

Example 2: Test method absorption velocity

The absorption velocity of a dressing is determined by the time necessary to completely absorb a test solution. Test solutions can be either saline solution (solution A) or exudate solution (solution B). Solutions (saline solution or exudates solution) as well as test products must be preconditioned at room temperature prior to testing by leaving the test products and the solutions for two hours at 22 °C.

A 50 mL buret is filled with the test solution. The liquid level should be 15 mL. The dressing is placed under the buret, so that the surface which in use faces the wound now faces the buret. The distance between the buret and the dressing is adjusted to 1 cm. The tap of the buret is opened, and simultaneously a stopwatch is started, while 2 mL of test solution are allowed to flow out. The stopwatch is stopped immediately when the 2 mL of test solution are completely absorbed by the dressing, i.e. when no drop is remaining over the wound contact layer. If the dressing is big enough, 1 to 3 measurements can be made on the same dressing.

Positions of the tests on a dressing are one in the centre, two further points along a diagonal in direction to the corners of the dressing, similar to the position of the three points on a regular die.

At least five samples must be investigated.

Example 3: Test method absorption capacity

Solutions (saline solution or exudates solution) as well as test products must be preconditioned at room temperature prior to testing by leaving the test products and the test solutions for two hours at 22 °C.

The basic mass ($m_1$) of a dressing is determined after removal of the release liners.

The length and width of the absorbent core is determined so that the absorbent core surface (S) can be determined. A bowl is filled with the test solution. The weight of testing solution should be at least 40 times higher than the dressing itself. The dressing is put into the bowl, and simultaneously a stopwatch is started. The side of the dressing which in use faces the wound side should face the bottom of the bowl. The back side of the dressing should be on the top. The dressing must not be glued to the bottom of the bowl. The dressing is left within the bowl for 30 min +/- 1 min. After 30 min, the dressing is removed from the bowl. The dressings may only be manipulated via the borders and not the absorbent core itself. The dressings are fixed on one corner to a stand with a clamp and allowed to hang for 20 min at room temperature. The wet mass ($m_2$) of the dressing is determined. The amount of liquid ($m_{liquid}$) absorbed is calculated:

$$m_{liquid} = m_2 - m_1$$

The absorption capacity is the amount of liquid absorbed ($m_2 - m_1$) by the absorbent core surface (S) and is given in g/ 100 cm$^2$.

$$\text{absorption capacity} = m_2 - m_1 / S \times 100.$$

## Brief Description of the Drawings

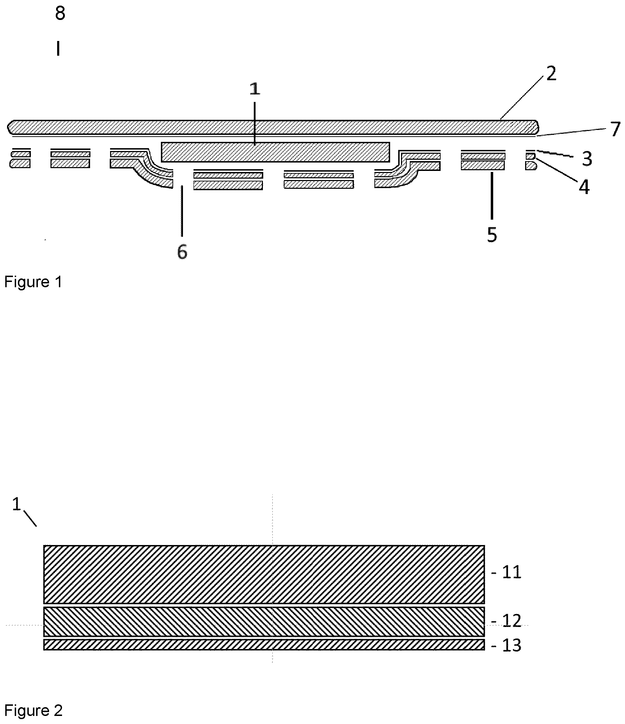

Fig.1 shows a cross-section of a preferred embodiment of a wound dressing 8 according to the invention. The dressing has a backing layer 2 comprising a nonwoven material. A layer 7 made of an adhesive acrylic is present on the proximate surface of the backing layer 2. A perforated atraumatic wound contact layer comprising a polyurethane layer 4, an adhesive acrylic layer 3 and an adhesive silicone layer 5 as skin adherent is present at the proximate surface of the dressing 8. The polyurethane layer 4 is located between the adhesive acrylic layer 3 and the adhesive silicone layer 5. The wound contact layer has a plurality of apertures 6, which are distributed over the wound contacting layer and which extend through all layers 5, 4, 3. The dressing further has an absorbent wound pad 1 comprising super absorbent fibers and a net layer 13 (not shown in Fig. 1, for details of wound pad 1 see Fig. 2), the net layer 13 being directed towards the wound side. Preferably, the wound pad 1 comprises a first nonwoven layer, wherein the superabsorbent fibers are integrated. Preferably, the wound pad further includes a second nonwoven layer, which is located between the first nonwoven layer and the net layer 13. The perforated atraumatic wound contact layer and the backing layer 2 extend beyond the wound pad 1 thereby creating an edge region. The backing layer 2 and the perforated atraumatic wound contact layer are bonded together at the edge region.

Fig. 2 shows a detailed cross-section of multi-layered wound pad 1, which can be component of a preferred embodiment of the invention. The wound pad 1 has three different layers 11, 12, 13 being directly connected to each other. At the proximal side of wound pad 1 a net layer 13 is disposed. Net layer 13 is made from a hydrophobic material such as polyethylene and has no absorbent properties. The first nonwoven layer 11 is the absorbent layer having the strongest absorption capacity of the wound pad 1. It is made of superabsorbent fibers mixed with fibers made from a blend of viscose, polyethylene and polypropylene. A second nonwoven layer 12 is sandwiched between the first nonwoven layer 11 and the net layer 13. The second nonwoven layer 12 serves as a transmission layer. It may for example consist of viscose, polyethylene and polypropylene. This layer 13 allows the wound exudate to be quickly absorbed into the wound pad. The layer 13 has some absorbent properties, but less than the distal layer 11.

**Claims**

1. A wound dressing (8) comprising

   - a backing layer (2) comprising a nonwoven material,
   - an absorbent wound pad (1) comprising super absorbent fibers and a net layer (13), the net layer (13) being directed towards the wound side,
   - a perforated atraumatic wound contact layer comprising a polyurethane layer (4), an adhesive acrylic layer (3) and an adhesive silicone layer (5) as skin adherent, wherein the polyurethane layer (4) is located between the adhesive acrylic layer (3) and the adhesive silicone layer (5) and

   wherein the perforated atraumatic wound contact layer and the backing layer (2) extend beyond the wound pad (1) thereby creating an edge region and wherein the backing layer (2) and the perforated atraumatic wound contact layer are bonded together at the edge region.

2. The wound dressing (8) according to claim 1, wherein the wound pad (1) comprises a first nonwoven layer (11) and the super absorbent fibers are integrated in this first nonwoven layer (11).

3. The wound dressing (8) according to claim 1 or 2, wherein the wound pad (1) comprises a second nonwoven layer (12) and wherein the second nonwoven layer (12) is located between the first nonwoven layer (11) and the net layer (13).

4. The wound dressing (8) according to claim 3, wherein the first (11) and the second nonwoven layers (12) are in direct contact with each other, so that no additional material is located between the first and the second nonwoven layer (12).

5. The wound dressing (8) according to claim 3 or 4, wherein the first and / or the second nonwoven layer (12) comprises one or more materials selected from the group consisting of: viscose fibers, polyethylene fibers and polypropylene fibers.

6. The wound dressing (8) according to one of the previous claims, wherein an additional layer of adhesive acrylic is present between the backing layer (2) and the wound pad (1), wherein the additional layer of adhesive acrylic optionally extends beyond the wound pad (1).

7. The wound dressing (8) according to one of the previous claims, wherein the perforations (6) of the wound contact layer completely penetrate the wound contact layer.

8. The wound dressing (8) according to one of the previous claims, wherein the layer of polyurethane is a polyurethane film.

9. The wound dressing (8) according to one of the previous claims, wherein the nonwoven backing material is hydrophobic and water-repellant.

10. The wound dressing (8) according to one of the previous claims, wherein the wound contact layer has an open area of between 5 % to 20 % of the entire area of the wound contact layer and / or wherein the wound contact layer comprises apertures having an essentially circular shape with an average diameter of between 0.1 mm to 2.8 mm.

11. The wound dressing (8) according to one of the previous claims, wherein the absorbent wound pad (1) has an absorption capacity of at least 20 g/100 cm$^2$.

12. The wound dressing (8) according to one of the previous claims, wherein the adhesive silicone layer (5) comprises silicone in an amount of from 90 g/m$^2$ to 180 g/m$^2$.

13. The wound dressing (8) according to one of the previous claims wherein the perforated atraumatic wound contact layer and the backing layer (2) extend in length and width beyond the wound pad (1) such that the edge region builds a continuous margin around the wound pad (1).

14. The wound dressing (8) according to one of the previous claims wherein the net layer (13) comprises polyethylene.

15. A method for manufacturing an absorbent wound dressing (8) according to claim 1 having an adhesive atraumatic wound contact layer, said method comprises the steps of:

    a) Providing a backing layer (2) comprising a nonwoven material
    b) Attaching an absorbent wound pad (1) comprising i) a layer having super absorbent fibers and ii) a net layer (13) to the backing layer (2), whereby the net layer (13) is directed towards the wound side,
    c) Providing a polyurethane layer (4) coated with an adhesive acrylic layer (7) on one side and coated with an adhesive silicone layer (5) on the other side wherein the polyurethane layer (4), the adhesive acrylic layer (7) and the adhesive silicone layer (5) are perforated and
    d) Attaching the polyurethane layer (4) with the adhesive acrylic layer (7) towards the backing layer (2) and the wound pad (1), whereby the adhesive silicone layer (5) is to be oriented in a way that it can be placed on a wound.

**Patentansprüche**

1. Wundverband (8) umfassend

    - eine Trägerschicht (2), die ein Vliesmaterial umfasst,
    - ein absorptionsfähiges Wundkissen (1), das superabsorbierende Fasern und eine Netzschicht (13) umfasst, wobei die Netzschicht (13) zu der Wundseite hin gerichtet ist,
    - eine perforierte atraumatische Wundkontaktschicht, die eine Polyurethanschicht (4), eine haftfähige Acrylschicht (3) und eine haftfähige Siliconschicht (5) als Hautkleber umfasst, wobei die Polyurethanschicht (4) zwischen der haftfähigen Acrylschicht (3) und der haftfähigen Siliconschicht (5) angeordnet ist und

    wobei sich die perforierte atraumatische Wundkontaktschicht und die Trägerschicht (2) über das Wundkissen (1) hinaus erstrecken, wodurch ein Randbereich entsteht, und wobei die Trägerschicht (2) und die perforierte atraumatische Wundkontaktschicht an dem Randbereich miteinander verbunden sind.

2. Wundverband (8) nach Anspruch 1, wobei das Wundkissen (1) eine erste Vliesschicht (11) umfasst und die superabsorbierenden Fasern in diese erste Vliesschicht (11) integriert sind.

3. Wundverband (8) nach Anspruch 1 oder 2, wobei das Wundkissen (1) eine zweite Vliesschicht (12) umfasst und wobei die zweite Vliesschicht (12) zwischen der ersten Vliesschicht (11) und der Netzschicht (13) angeordnet ist.

4. Wundverband (8) nach Anspruch 3, wobei sich die erste (11) und die zweite Vliesschicht (12) in direktem Kontakt miteinander befinden, so dass kein zusätzliches Material zwischen der ersten und der zweiten Vliesschicht (12) angeordnet ist.

5. Wundverband (8) nach Anspruch 3 oder 4, wobei die erste und/oder die zweite Vliesschicht (12) ein oder mehrere Materialien ausgewählt aus der Gruppe bestehend aus: Viskosefasern, Polyethylenfasern und Polypropylenfasern umfassen.

6. Wundverband (8) nach einem der vorstehenden Ansprüche, wobei zwischen der Trägerschicht (2) und dem Wundkissen (1) eine zusätzliche Schicht aus haftfähigem Acryl vorhanden ist, wobei sich die zusätzliche Schicht aus haftfähigem Acryl gegebenenfalls über das Wundkissen (1) hinaus erstreckt.

7. Wundverband (8) nach einem der vorstehenden Ansprüche, wobei die Perforationen (6) der Wundkontaktschicht die Wundkontaktschicht vollständig durchdringen.

8. Wundverband (8) nach einem der vorstehenden Ansprüche, wobei die Schicht aus Polyurethan eine Polyurethanfolie ist.

9. Wundverband (8) nach einem der vorstehenden Ansprüche, wobei das Vlies-Trägermaterial hydrophob und wasserabweisend ist.

10. Wundverband (8) nach einem der vorstehenden Ansprüche, wobei die Wundkontaktschicht eine offene Fläche zwischen 5 % bis 20 % der gesamten Fläche der Wundkontaktschicht aufweist und/oder wobei die Wundkontaktschicht Öffnungen mit einer im Wesentlichen kreisförmigen Form mit einem mittleren Durchmesser von zwischen 0,1 mm und 2,8 mm umfasst.

11. Wundverband (8) nach einem der vorstehenden Ansprüche, wobei das absorptionsfähige Wundkissen (1) eine Absorptionskapazität von wenigstens 20 g/100 cm$^2$ aufweist.

12. Wundverband (8) nach einem der vorstehenden Ansprüche, wobei die haftfähige Siliconschicht (5) Silicon in einer Menge von 90 g/m$^2$ bis 180 g/m$^2$ umfasst.

13. Wundverband (8) nach einem der vorstehenden Ansprüche, wobei sich die perforierte atraumatische Wundkontaktschicht und die Trägerschicht (2) in Länge und Breite über das Wundkissen (1) hinaus erstrecken, so dass der Randbereich einen durchgehenden Rand um das Wundkissen (1) bildet.

14. Wundverband (8) nach einem der vorstehenden Ansprüche, wobei die Netzschicht (13) Polyethylen umfasst.

15. Verfahren zur Herstellung eines absorptionsfähigen Wundverbands (8) nach Anspruch 1 mit einer haftfähigen atraumatischen Wundkontaktschicht, wobei das Verfahren die Schritte umfasst:

    a) Bereitstellen einer Trägerschicht (2), die ein Vliesmaterial umfasst
    b) Anbringen eines absorptionsfähigen Wundkissens (1), das i) eine Schicht mit superabsorbierenden Fasern und ii) eine Netzschicht (13) umfasst, an der Trägerschicht (2), wobei die Netzschicht (13) zu der Wundseite hin gerichtet ist,

c) Bereitstellen einer Polyurethanschicht (4), die auf einer Seite mit einer haftfähigen Acrylschicht (7) beschichtet ist und auf der anderen Seite mit einer haftfähigen Siliconschicht (5) beschichtet ist, wobei die Polyurethanschicht (4), die haftfähige Acrylschicht (7) und die haftfähige Siliconschicht (5) perforiert sind, und

d) Anbringen der Polyurethanschicht (4) mit der haftfähigen Acrylschicht (7) in Richtung zu der Trägerschicht (2) und dem Wundkissen (1), wobei die haftfähige Siliconschicht (5) so auszurichten ist, dass sie auf eine Wunde platziert werden kann.

## Revendications

1. Pansement (8) comprenant

   - une couche de support (2) comprenant un matériau non tissé,
   - une compresse absorbante (1) comprenant des fibres superabsorbantes et une couche de tulle (13), la couche de tulle (13) étant dirigée vers le côté de la plaie,
   - une couche de contact avec la plaie atraumatique perforée comprenant une couche de polyuréthane (4), une couche d'acrylique adhésif (3) et une couche de silicone adhésive (5) pour adhérer à la peau, dans lequel la couche de polyuréthane (4) est située entre la couche d'acrylique adhésif (3) et la couche de silicone adhésive (5) et

   dans lequel la couche de contact avec la plaie atraumatique perforée et la couche de support (2) s'étendent au-delà de la compresse (1), créant ainsi une région de bord, et dans lequel la couche de support (2) et la couche de contact avec la plaie atraumatique perforée sont reliées au niveau de la région de bord.

2. Pansement (8) selon la revendication 1, dans lequel la compresse (1) comprend une première couche de non-tissé (11) et les fibres superabsorbantes sont intégrées dans cette première couche de non-tissé (11).

3. Pansement (8) selon la revendication 1 ou 2, dans lequel la compresse (1) comprend une deuxième couche de non-tissé (12) et dans lequel la deuxième couche de non-tissé (12) est située entre la première couche de non-tissé (11) et la couche de tulle (13).

4. Pansement (8) selon la revendication 3, dans lequel la première (11) et la deuxième couche de non-tissé (12) sont en contact direct l'une avec l'autre, de sorte qu'aucun matériau supplémentaire ne se situe entre la première et la deuxième couche de non-tissé (12).

5. Pansement (8) selon la revendication 3 ou 4, dans lequel la première et/ou la deuxième couche de non-tissé (12) comprennent un ou plusieurs matériaux choisis dans le groupe constitué par : les fibres de viscose, les fibres de polyéthylène et les fibres de polypropylène.

6. Pansement (8) selon une des revendications précédentes, dans lequel une couche supplémentaire d'acrylique adhésif est présente entre la couche de support (2) et la compresse (1), la couche supplémentaire d'acrylique adhésif s'étendant éventuellement au-delà de la compresse (1).

7. Pansement (8) selon une des revendications précédentes, dans lequel les perforations (6) de la couche de contact avec la plaie pénètrent entièrement la couche de contact avec la plaie.

8. Pansement (8) selon une des revendications précédentes, dans lequel la couche de polyuréthane est un film de polyuréthane.

9. Pansement (8) selon une des revendications précédentes, dans lequel le matériau de support non tissé est hydrophobe et étanche à l'eau.

10. Pansement (8) selon une des revendications précédentes, dans lequel la couche de contact avec la plaie a une surface ouverte représentant entre 5 % et 20 % de la surface totale de la couche de contact avec la plaie et/ou

    dans lequel la couche de contact avec la plaie comprend des ouvertures ayant une forme essentiellement circulaire avec un diamètre moyen compris entre 0,1 mm et 2,8 mm.

11. Pansement (8) selon une des revendications précédentes, dans lequel la compresse absorbante (1) possède une capacité d'absorption d'au moins 20 g/100 cm$^2$.

12. Pansement (8) selon une des revendications précédentes, dans lequel la couche de silicone adhésive (5) comprend de la silicone dans une quantité allant de 90 g/m$^2$ à 180 g/m$^2$.

13. Pansement (8) selon une des revendications précédentes, dans lequel la couche de contact avec la plaie atraumatique perforée et la couche de support (2) s'étendent en longueur et en largeur au-delà de la compresse (1), de sorte que la région de bord constitue une marge continue autour de la compresse (1).

14. Pansement (8) selon une des revendications précédentes, dans lequel la couche de tulle (13) comprend du polyéthylène.

15. Procédé de fabrication d'un pansement absorbant (8) selon la revendication 1 comportant une couche de contact avec la plaie atraumatique adhésive, ledit procédé comprenant les étapes suivantes :

a) obtention d'une couche de support (2) comprenant un matériau non tissé,

b) fixation d'une compresse absorbante (1) comprenant i) une couche comportant des fibres superabsorbantes et ii) une couche de tulle (13) à la couche de support (2), la couche de tulle (13) étant dirigée vers le côté de la plaie,

c) obtention d'une couche de polyuréthane (4) revêtue d'une couche d'acrylique adhésif (7) sur un côté et revêtue d'une couche de silicone adhésive (5) sur l'autre côté, la couche de polyuréthane (4), la couche d'acrylique adhésif (7) et la couche de silicone adhésive (5) étant perforées, et

d) fixation de la couche de polyuréthane (4) avec la couche d'acrylique adhésif (7) vers la couche de support (2) et la compresse (1), moyennant quoi la couche de silicone adhésive (5) doit être orientée de manière à ce qu'elle puisse être placée sur une plaie.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3281617 A1
- EP 3669843 A1
- WO 9319710 A1
- GB 1280631 A